(19) Europäisches Patentamt
European Patent Office
Office européen des brevets

(11) Veröffentlichungsnummer: **0 222 217 B1**

(12) **EUROPÄISCHE PATENTSCHRIFT**

(45) Veröffentlichungstag der Patentschrift: **06.11.91**

(51) Int. Cl.5: **C07C 49/84**, C07C 49/255, C07C 321/06, C07C 49/175, C07C 45/64

(21) Anmeldenummer: **86114627.2**

(22) Anmeldetag: **22.10.86**

Die Akte enthält technische Angaben, die nach dem Eingang der Anmeldung eingereicht wurden und die nicht in dieser Patentschrift enthalten sind.

# (54) Dialkoxyketone und ein Verfahren zu ihrer Herstellung.

(30) Priorität: **08.11.85 DE 3539629**

(43) Veröffentlichungstag der Anmeldung:
**20.05.87 Patentblatt 87/21**

(45) Bekanntmachung des Hinweises auf die Patenterteilung:
**06.11.91 Patentblatt 91/45**

(84) Benannte Vertragsstaaten:
**AT BE CH DE FR GB IT LI NL SE**

(56) Entgegenhaltungen:
**DE-A- 2 730 462**

**CHEMICAL ABSTRACTS, Band 98, Juni 1983, Seite 79, Spalte 1, Zusammenfassungsnr. 217105w, Columbus, Ohio, US; & JP - A - 57 210 076 (SUMITOMO CHEMICAL CO., IPPOSHA OIL INDUSTRIES CO.) veröffentlicht am 23.12.1982**

**JERRY MARCH "Advanced organic chemistry, reactions, mechanisms and structure", 2. Auflage, 1977, International Student Edition, McGraw Hill Kogakusha, Ltd.**

(73) Patentinhaber: **BASF Aktiengesellschaft**
**Carl-Bosch-Strasse 38**
**W-6700 Ludwigshafen(DE)**

(72) Erfinder: **Goetz, Norbert, Dr.**
**Schoefferstrasse 25**
**W-6520 Worms 1(DE)**
Erfinder: **Sproesser, Linhard, Dr.**
**Goethestrasse 4**
**W-6702 Bad Duerkheim(DE)**
Erfinder: **Buschmann, Ernst, Dr.**
**Georg-Ludwig-Krebs-Strasse 10**
**W-6700 Ludwigshafen(DE)**
Erfinder: **Karbach, Stefan, Dr.**
**Sperlinggasse 3**
**W-6700 Ludwigshafen(DE)**

**EP 0 222 217 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von neuen Dialkoxyketonen der Formel I

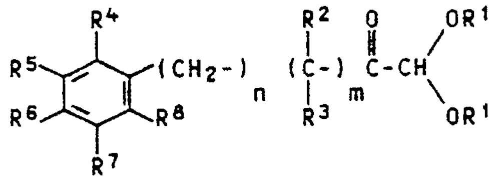

I

in der

| | |
|---|---|
| $R^1$ | $C_1$-$C_{13}$-Alkyl, |
| $R^2$, $R^3$ | Wasserstoff oder $C_1$-$C_4$-Alkyl, |
| $R^4$-$R^8$ | Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Halogenalkoxy, $C_1$-$C_4$-Halogenalkylthio, Phenyl, das gegebenenfalls durch Halogen, $C_1$-$C_4$-Alkyl oder $C_1$-$C_4$-Alkoxy substituiert ist; oder zwei benachbarte Reste $R^4$ bis $R^8$ eine $C_3$-$C_5$-Alkylenkette, die gegebenenfalls durch $C_1$-$C_4$-Alkyl substituiert ist, oder einen gegebenenfalls substituierten annellierten aromatischen Ring und |
| m,n | 0 oder 1 bedeuten, |

mit der Maßgabe, daß m und n nicht für 0 stehen, wenn jeweils $R^4$ oder $R^8$ $C_1$-$C_4$-Alkoxy; $R^5$ oder $R^7$ $C_1$-$C_4$-Alkylthio; $R^6$ Halogen, $C_1$-$C_4$-Alkyl, $C_1$-$C_4$-Alkoxy, Phenyl; $R^5$ und $R^6$ oder $R^6$ und $R^7$ jeweils $C_1$-$C_4$-Alkoxy; $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten. Insbesondere sind solche Dialkoxyketone I ausgenommen, bei denen m und n für 0 stehen, wenn einer der Reste $R^4$ bis $R^8$ $C_1$-$C_4$-Alkylthio, oder jeweils bis zu drei der Reste $R^4$ bis $R^8$ Halogen, $C_1$-$C_4$-Alkyl, $C_1$-$C_4$-Alkoxy oder Phenyl, oder alle Reste $R^4$ bis $R^8$ Wasserstoff bedeuten.

Einige $\omega,\omega$-Dialkoxyacetophenone, die im Phenylring unsubstituiert oder durch Alkyl, Alkoxy, Alkylthio, Halogen oder Phenyl speziell substituiert sind, sind bekannt (JP-A-210 076/1982. US-A-3 409 674, US-A-4 130 661, DE-A-2 730 462).

Aus "Advanced Organic Chemistry" von Jerry March, 2. Auflage 1977, S. 810 ist die Synthese von Acetalen und Ketalen aus Aldehyden bzw. Ketonen und Alkoholen in Gegenwart eines sauren Katalysators bekannt. Auf Seite 1101 werden Verfahren zur Herstellung von $\alpha$-Ketoaldehyden und $\alpha$-Diketonen durch Oxidation von $\alpha$-Methyl- oder $\alpha$-Methylenketonen aufgezeigt. Als Oxidationsmittel wird u.a. Distickstofftrioxid erwähnt, besonders aber wird Selendioxid empfohlen.

Aufgabe der Erfindung war es, ein technisch gut durchführbares Verfahren zur Herstellung der neuen Dialkoxyketone bereitzustellen.

Demgemäß wurde ein Verfahren zur Herstellung der Dialkoxyketone der Formel I gefunden, welches dadurch gekennzeichnet ist, daß man ein Keton der Formel II

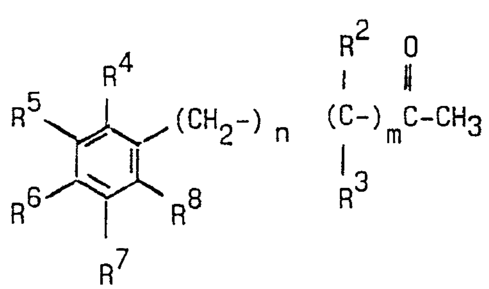

II

mit Distickstofftrioxid in Gegenwart von überschüssigem Alkohol der Formel $R^1$-OH und in Gegenwart eines sauren Katalysators bei einer Temperatur von 0 bis 140 °C behandelt.

Die in den Resten $R^1$, $R^2$, $R^3$, $R^4$, $R^5$, $R^6$, $R^7$ und $R^8$ aufgeführten Alkylgruppen können jeweils geradkettig oder verzweigt sein.

$R^1$ in Formel I bedeutet $C_1$-$C_{13}$-Alkyl, z.B. Methyl, Ethyl, Propyl, Isopropyl, Butyl, Isobutyl, tert-Butyl, Pentyl, 2-Methylbutyl, Hexyl, 2-Methylpentyl, Heptyl, Octyl, 2-Ethylhexyl, Isooctyl, Nonyl, Isononyl, Decyl, Isodecyl, 3,5,6,7-Tetramethylnonyl oder Isotridecyl. (Die Bezeichnungen Isooctyl, Isononyl, Isodecyl und

Isotridecyl sind Trivialbezeichnungen und stammen von den nach der Oxosynthese erhaltenen Alkoholen (vgl. dazu Ullmann, Enzyklopädie der Technischen Chemie, 4. Auflage, Band 7, Seiten 216 und 217 sowie Band 11, Seiten 435 und 436). Bevorzugt sind $C_1$-$C_5$-Alkyl, besonders bevorzugt ist Methyl.

$R^2$ und $R^3$, die gleich oder verschieden sind, bedeuten in Formel I unabhängig voneinander beispielsweise Wasserstoff, Methyl, Ethyl, Propyl, Isopropyl, Butyl, Isobutyl oder tert-Butyl.

$R^4$, $R^5$, $R^6$, $R^7$ und $R^8$, die gleich oder verschieden sind bedeuten in Formel I unabhängig voneinander beispielsweise Wasserstoff, Methyl, Ethyl, Propyl, Isopropyl, Butyl oder Isobutyl; Fluor, Chlor oder Brom; Chlormethyl, Difluormethyl, Trifluormethyl, Pentafluorethyl, 2-Chlor-1,1,2,2-tetrafluorethyloder Nonafluorbutyl; Methoxy, Ethoxy, Propoxy, Isopropoxy, Butoxy oder Isobutoxy; Methylthio, Ethylthio, Propylthio, Isopropylthio, Butylthio oder Isobutylthio; Chlormethoxy, Difluormethoxy, Trifluormethoxy, Trichlormethoxy, Pentafluorethoxy, 2-Chlor-1,1,2,2-tetrafluorethoxy oder Nonafluorbutoxy; Chlormethylthio, Difluormethylthio, Trifluormethylthio, Trichlormethylthio, Pentafluorethylthio, 2-Chlor-1,1,2,2-tetrafluorethylthio oder Nonafluorbutylthiophenyl, 4-Chlorphenyl, 2-Chlorphenyl, 2-Methylphenyl, 4-Methylphenyl, 2-Methoxyphenyl oder 4-Ethoxyphenyl. Bevorzugt sind Fluor, Chlor, Brom, Trifluormethyl, Trifluormethoxy, sowie Wasserstoff mit der Maßgabe, daß m und/oder n ungleich 0 sind.

Weiterhin können jeweils zwei benachbarte Reste der Reste $R^4$ bis $R^8$ in Formel I zusammen beispielsweise eine 1,3-Propylen-, 1,4-Butylen-, 1,5-Pentylen- oder 1,1,4,4-Tetramethyl-1,4-butylenkette bedeuten. Bevorzugt ist die 1,1,4,4-Tetramethyl-1,4-butylenkette. Schließlich können auch jeweils zwei benachbarte Reste der Reste $R^4$ bis $R^8$ in Formel I zusammen einen gegebenenfalls substituierten annellierten aromatischen Ring, bevorzugt einen annellierten Benzolring, ausbilden.

Beispielhaft seien für den Phenylrest der Formel III in Formel I

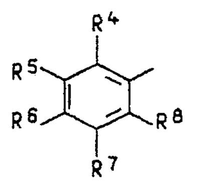

(III)

folgende Reste genannt: 2-Chlorphenyl, 3-Fluorphenyl, 3-Brom-4-fluorphenyl,3,4-Dichlorphenyl, 2,-Dichlorphenyl, 2,4-Difluorphenyl, 2-Methylphenyl, 4-tert-Butylphenyl, 4-Trifluormethylphenyl, 2-Methyl-4-trifluormethylphenyl, 2-Ethoxyphenyl, 4-Methylthiophenyl, 4-Trifluormethoxyphenyl, 4-Trifluormethylthiophenyl, 2-Naphthyl oder 5,5,8,8-Tetramethyl-tetrahydronaphthyl.

Besonders bevorzugt sind solche Verbindungen der Formel I, in denen $R^4$ und $R^6$ bzw. $R^6$ und $R^8$ Chlor, die übrigen Reste am Phenylring Wasserstoff und m und n 0 bedeuten.

Zur Durchführung des erfindungsgemäßen Verfahrens werden Ketone der Formel II

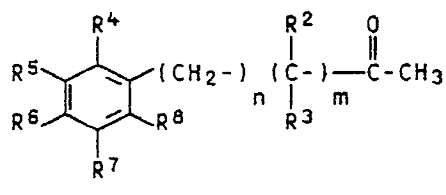

II

mit Distickstofftrioxid in Gegenwart von überschüssigem Alkohol der Formel $R^1$-OH, und in Gegenwart eines sauren Katalysators bei einer Temperatur von 0 bis 140 °C, vorzugsweise 20 bis 80 °C behandelt.

Das erfindungsgemäße Verfahren wird üblicherweise bei Normaldruck durchgeführt. In einigen Fällen kann es jedoch von Vorteil sein, auch unter erhöhtem Druck (1 bis 10 bar) zu arbeiten.

Als saure Katalysatoren kommen starke Protonensäuren oder Lewis-Säuren, z.B. Fluorwasserstoff, Chlorwasserstoff oder Bortrifluorid in Betracht. Die Verwendung von Schwefelsäure hat sich als weniger günstig erwiesen.

Pro Mol Keton werden 0,05 bis 1 Mol, vorzugsweise 0,2 bis 0,5 Mol saurer Katalysator zugesetzt.

Zweckmäßig erzeugt man das ebenfalls an der Umsetzung beteiligte Distickstofftrioxid in situ durch eine vorgelagerte Gasreaktion aus Stickstoffmonoxid und Sauerstoff:

$2\ NO + 1/2\ O_2 \rightarrow N_2O_3$.

Der Alkohol $R^1OH$ wird im Überschuß angewandt, da er einerseits als Reaktionspartner auftritt und andererseits auch als Reaktionsmedium dienen soll.

In einer bevorzugten Ausführungsform legt man das Keton II, den Alkohol $R^1$-OH sowie den sauren Katalysator vor und beginnt im oben genannten Temperaturbereich mit der Zuführung von Stickstoffmonoxid und Sauerstoff im Molverhältnis 4:1.

Die Zuführung von Stickstoffmonoxid und Sauerstoff erfolgt vorteilhaft durch zwei koaxiale Rohre, die in das Reaktionsmedium eintauchen. Beim Austritt aus den koaxialen Rohren setzt die Gasreaktion ein, wobei sich Distickstofftrioxid bildet, das über ein intermediär entstehendes Alkylnitrit der Formel $R^1ONO$ mit dem Keton zum Zielprodukt reagiert.

Beim Arbeiten mit niederen Alkoholen $R^1$-OH (z.B. Methanol oder Ethanol) empfiehlt es sich, ein wirkungsvolles Kühlsystem (z.B. einen Trockeneiskühler) in der Apparatur vorzusehen, um das intermediär gebildete, leicht flüchtige niedere Alkylnitrit zu kondensieren. Dies ist sowohl aus gesundheitlichen Gründen wie auch im Sinne eines raschen Reaktionsablaufs erforderlich. Ein Verlust an Alkylnitrit durch Verdampfen hat schließlich entsprechend längeres Eingasen von Stickstoffmonoxid und Sauerstoff zur Folge.

Nach beendeter Umsetzung, die im allgemeinen 1 bis 10 Stunden in Anspruch nimmt, wird durch Zugabe von Basen (z.B. Natriumhydroxid, Kaliumhydroxid, Kaliumcarbonat oder Natriumalkanolat) ein pH-Bereich von 7 bis 10 eingestellt. Dabei wird auch überschüssiges Alkylnitrit zerstört.

Im Falle der leicht flüchtigen niederen Alkylnitrite kann deren Vernichtung auch mittels eines Gaswaschturms, der beispielsweise Natriumhydroxid enthält, und durch den das jeweilige Alkylnitrit hindurchgeleitet wird, geschehen.

Für den Fall, daß die Zielprodukte im jeweiligen Alkohol schwer löslich sind, werden sie abgetrennt, gewaschen und getrocknet und gegebenenfalls umkristallisiert. Sind die Zielprodukte im jeweiligen Alkohol jedoch löslich, so wird dieser unter vermindertem Druck entfernt und der Rückstand wird in einem organischen Lösungsmittel. z.B. Dichlormethan oder Toluol, aufgenommen, mit Wasser gewaschen und getrocknet. Nach Entfernen des Lösungsmittels können auch hier die anfallenden Zielprodukte gegebenenfalls weiter gereinigt werden.

Das erfindungsgemäße Verfahren kann sowohl in kontinuierlicher wie auch in diskontinuierlicher Arbeitsweise durchgeführt werden.

Für den Fall, daß die Umsetzung mit höheren Alkoholen vorgesehen ist, kann man prinzipiell auch das erfindungsgemäße Verfahren zunächst mit niederen Alkoholen durchführen und die resultierenden Dialkoxyketone in Gegenwart der betreffenden höheren Alkohole und einer Säure einer Umacetalisierungsreaktion unterwerfen.

Die neuen Dialkoxyketone der Formel I sind wertvolle Zwischenprodukte für die Synthese von Pflanzenschutzmitteln, insbesondere von Fungiziden und Bioregulatoren, wie sie z.B. in der DE-A-2 926 280, DE-A-3 047 726 und DE-A-3 150 204 genannt sind.

## Herstellungsbeispiele

### Beispiel 1

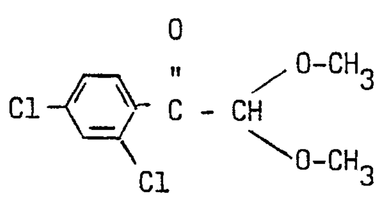

Herstellung von 2,4-Dichlor-α,α-dimethoxyacetophenon

In einem Reaktionsgefäß, das mit einem solekühlbaren Rückflußkühler und einem dahintergeschalteten Trockeneiskühler ausgerüstet war, wurden 9,54 Gewichtsteile 2,4-Dichloracetophenon und 19,19 Gewichtsteile Methanol vorgelegt. Es wurde 1 Gewichtsteil Chlorwasserstoff in die Lösung eingegast und anschließend bis zum Rückfluß erhitzt. Durch zwei koaxiale Rohre wurden nun bei einer Temperatur von 65° C 7 Stunden lang unter Rühren 0,55 Gewichtsteile/Stunde Stickstoffmonoxid und 0,15 Gewichtsteile/ Stunde Sauerstoff in die Lösung eingegast. Die Zufuhr von Sauerstoff erfolgte dabei durch das innere Rohr. Nach Beendigung der Gaszufuhr wurde das Reaktionsgemisch unter schwachem Stickstoffstrom (Zufuhr durch das innere Rohr) 1 Stunde unter Rückfluß nachgerührt. Anschließend wurde unter Kühlen solange 10 gew.%ige Natronlauge zugeführt, bis sich ein konstanter pH-Wert von 9 einstellte. Nach dem Abkühlen des Reaktionsgemisches auf 0° C wurde abgesaugt und der Rückstand mit Wasser gewaschen und getrocknet.

Ausbeute: 9,34 Gewichtsteile (75 % d.Th.) Fp. : 47° C.

Die in der nachfolgenden Tabelle aufgeführten Verbindungen können in analoger Weise erhalten werden.

| Verbindung Nr. | 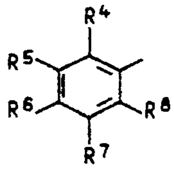 | n | m | $R^3$ | $R^2$ | $R^1$ | Phys. Daten |
|---|---|---|---|---|---|---|---|
| 2 | 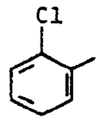 | 0 | 0 | - | - | $CH_3$ | 90°C/0.3 |
| 3 | 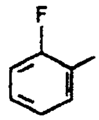 | 0 | 0 | - | - | $CH_3$ | 73°C/0,3 |
| 4 | 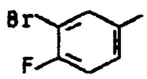 | 0 | 0 | - | - | $CH_3$ | 131°C/0,3 |
| 5 | 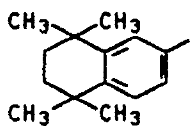 | 0 | 0 | - | - | $CH_3$ | 157°C/0,5 |
| 6 | 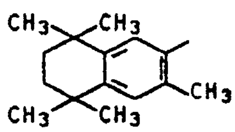 | 0 | 0 | - | - | $CH_3$ | 167°C/0,4 |
| 7 | 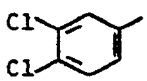 | 0 | 0 | - | - | $CH_3$ | |
| 8 | 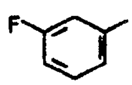 | 0 | 0 | - | - | $CH_3$ | |
| 9 | 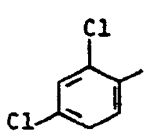 | 0 | 0 | - | - | iso-$C_4H_9$ | |
| 10 | 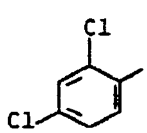 | 0 | 0 | - | - | $C_5H_{11}$ | 130-5°C/0.3 |

| Verbindung Nr. | 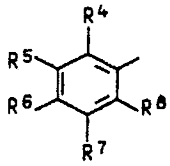 | n | m | $R^3$ | $R^2$ | $R^1$ | Phys. Daten |
|---|---|---|---|---|---|---|---|
| 11 | 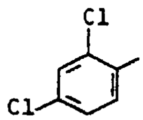 | 0 | 0 | - | - | $C_2H_5$ | Kp.114-9/0,2 |
| 12 | 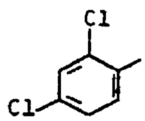 | 0 | 0 | - | - | $C_3H_7$ | Kp.148-52°C/ 0,8 |
| 13 | 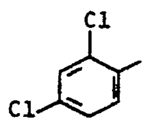 | 0 | 0 | - | - | $C_4H_9$ | Kp.172-4°C/2 |
| 14 | 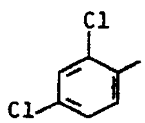 | 0 | 0 | - | - | 3-Methyl-butyl | Kp.172-82/ 0,5 |
| 15 | 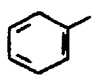 | 1 | 1 | $CH_3$ | $CH_3$ | $CH_3$ | 97-101°C/ 0,3 |
| 16 | 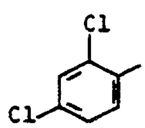 | 1 | 1 | $CH_3$ | $CH_3$ | $CH_3$ | 145-50°C/1 |
| 17 | 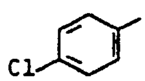 | 1 | 1 | $CH_3$ | $CH_3$ | $CH_3$ | 155-8°C/0,4 |
| 18 | 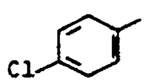 | 1 | 1 | $CH_3$ | $CH_3$ | $C_3H_7$ | 175-80°C/ 0,5 |
| 19 | 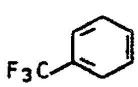 | 0 | 0 | - | - | $CH_3$ | |
| 20 | 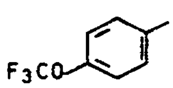 | 0 | 0 | - | - | $CH_3$ | |

| Verbindung Nr. | 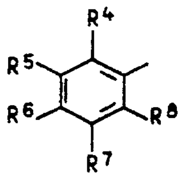 | n | m | $R^3$ | $R^2$ | $R^1$ | Phys. Daten |
|---|---|---|---|---|---|---|---|
| 21 | 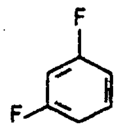 | 0 | 0 | - | - | $CH_3$ | |
| 22 | 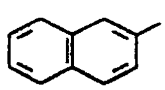 | 0 | 0 | - | - | $CH_3$ | |
| 23 | 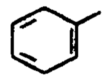 | 1 | 1 | $CH_3$ | $CH_3$ | $CH_3$ | |

## Patentansprüche

1. Verfahren zur Herstellung von Dialkoxyketonen der Formel I

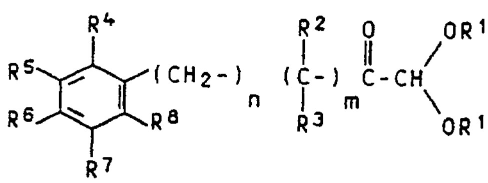

I

in der

| | |
|---|---|
| $R^1$ | $C_1$-$C_{13}$-Alkyl, |
| $R^2$, $R^3$ | Wasserstoff oder $C_1$-$C_4$-Alkyl, |
| $R^4$ - $R^8$ | Wasserstoff, Halogen, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Halogenalkoxy, $C_1$-$C_4$-Halogenalkylthio, Phenyl, das gegebenenfalls durch Halogen, $C_1$-$C_4$-Alkyl oder $C_1$-$C_4$-Alkoxy substituiert ist; oder zwei benachbarte Reste $R^4$ bis $R^8$ eine $C_3$-$C_5$-Alkylenkette, die gegebenenfalls durch $C_1$-$C_4$-Alkyl substituiert ist, oder einen gegebenenfalls substituierten annellierten aromatischen Ring und |
| m, n | 0 oder 1 bedeuten, |

mit der Maßgabe, daß m und n nicht für 0 stehen, wenn jeweils $R^4$ oder $R^8$ $C_1$-$C_4$-Alkoxy; $R^5$ oder $R^7$ $C_1$-$C_4$-Alkylthio; $R^6$ Halogen, $C_1$-$C_4$-Alkyl, $C_1$-$C_4$-Alkoxy, Phenyl; $R^5$ und $R^6$ oder $R^6$ und $R^7$ jeweils $C_1$-$C_4$-Alkoxy; $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten, dadurch gekennzeichnet, daß man ein Keton der Formel II

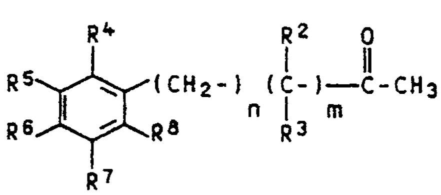

II

mit Distickstofftrioxid in Gegenwart von überschrüssigem Alkohol der Formel $R^1$-OH und in Gegenwart eines sauren Katalysators bei einer Temperatur von 0 bis 140 °C behandelt.

2. Verfahren zur Herstellung von Dialkoxyketonen der Formel I nach Anspruch 1, in der

| | |
|---|---|
| $R^1$ | $C_1$-$C_5$-Alkyl, |
| $R^2$, $R^3$ | Wasserstoff oder $C_1$-$C_4$-Alkyl, |
| $R^4$-$R^8$ | Wasserstoff, Fluor, Chlor, Brom, $C_1$-$C_4$-Alkyl, $C_1$-$C_4$-Fluoralkyl, $C_1$-$C_4$-Chloralkyl, $C_1$-$C_4$-Chlor-fluor-alkyl, $C_1$-$C_4$-Alkoxy, $C_1$-$C_4$-Alkylthio, $C_1$-$C_4$-Fluoralkoxy, $C_1$-$C_4$-Chloralkoxy, $C_1$-$C_4$-Chlor-fluor-alkoxy, $C_1$-$C_4$-Fluoralkylthio, $C_1$-$C_4$-Chloralkylthio, $C_1$-$C_4$-Chlor-fluor-alkylthio, Chlorphenyl, Methylphenyl, $C_1$- und $C_2$-Alkoxyphenyl, oder zwei benachbarte Reste $R^4$ bis $R^8$ eine $C_3$-$C_5$-Alkylenkette, die gegebenenfalls durch Methyl substituiert ist, oder einen gegebenenfalls durch Halogen und/oder $C_1$-$C_4$-Alkyl und/oder Trifluormethyl und/oder $C_1$-$C_2$-Alkoxy und/oder Methylthio und/oder Trifluormethoxy und/oder Trifluormethylthio substituierten annellierten Ring und |
| m, n | 0 oder 1 bedeuten, |

mit der Maßgabe, daß m und n nicht für 0 stehen, wenn jeweils $R^4$ oder $R^8$ $C_1$-$C_4$-Alkoxy; $R^5$ oder $R^7$ $C_1$-$C_4$-Alkylthio; $R^6$ Halogen, $C_1$-$C_4$-Alkyl, $C_1$-$C_4$-Alkoxy, Phenyl; $R^5$ und $R^6$ oder $R^6$ und $R^7$ jeweils $C_1$-$C_4$-Alkoxy; $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten, dadurch gekennzeichnet, daß man ein Keton der Formel II

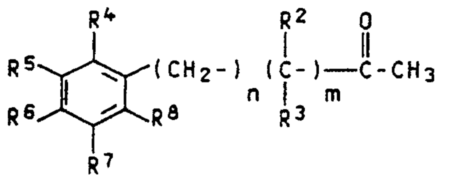

II

mit Distickstofftrioxid in Gegenwart von überschüssigem Alkohol der Formel $R^1$-OH und in Gegenwart eines sauren Katalysators bei einer Temperatur von 0 bis 140 °C behandelt.

3. Verfahren zur Herstellung von Dialkoxyketonen der Formel I nach Anspruch 1, in der

| | |
|---|---|
| $R^1$ | $C_1$-$C_5$-Alkyl, |
| $R^2$, $R^3$ | Wasserstoff oder $C_1$-$C_4$-Alkyl, |
| $R^4$-$R^8$ | Wasserstoff, Fluor, Chlor, Brom, $C_1$-$C_4$-Alkyl, $C_1$-$C_4$-Fluoralkyl, $C_1$-$C_4$-Chloralkyl, $C_1$-$C_4$-Chlor-fluor-alkyl, $C_1$-$C_4$-Alkoxy, $C_1$-$C_4$-Alkylthio, $C_1$-$C_4$-Fluoralkoxy, $C_1$-$C_4$-Chloralkoxy, $C_1$-$C_4$-Chlor-fluor-alkoxy, $C_1$-$C_4$-Fluoralkylthio, $C_1$-$C_4$-Chloralkylthio, $C_1$-$C_4$-Chlor-fluor-alkylthio, Chlorphenyl, Methylphenyl, $C_1$- und $C_2$-Alkoxyphenyl, oder zwei benachbarte Reste $R^4$ bis $R^8$ eine $C_3$-$C_5$-Alkylenkette, die gegebenenfalls durch Methyl substituiert ist, oder einen gegebenenfalls durch Halogen und/oder $C_1$-$C_4$-Alkyl und/oder Trifluormethyl und/oder $C_1$-$C_2$-Alkoxy und/oder Methylthio und/oder Trifluormethoxy und/oder Trifluormethylthio substituierten Phenyl oder annellierten Benzolring und |
| m, n | O oder 1 bedeuten, |

mit der Maßgabe, daß m und n nicht für 0 stehen, wenn jeweils $R^4$ oder $R^8$ $C_1$-$C_4$-Alkoxy; $R^5$ oder $R^7$ $C_1$-C-Alkylthio; $R^6$ Halogen, $C_1$-$C_4$-Alkyl, $C_1$-$C_4$-Alkoxy, Phenyl; $R^5$ und $R^6$ oder $R^6$ und $R^7$ jeweils $C_1$-$C_4$-Alkoxy; $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten, dadurch gekennzeichnet, daß man ein Keton der Formel II

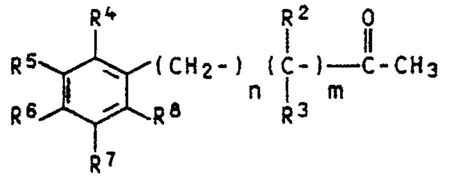 II

mit Distickstofftrioxid in Gegenwart von überschüssigem Alkohol der Formel $R^1$-OH und in Gegenwart eines sauren Katalysators bei einer Temperatur von 0 bis 140 °C behandelt.

**4.** Verfahren zur Herstellung von Dialkoxyketonen der Formel I nach Anspruch 1, in der

| | |
|---|---|
| $R^1$ | $C_1$-$C_5$-Alkyl, |
| $R^2$, $R^3$ | Wasserstof oder $C_1$-$C_4$-Alkyl, |
| $R^4$-$R^8$ | Wasserstoff, Fluor, Chlor, Brom, Trifluormethyl, Trifluormethoxy oder zwei benachbarte Reste $R^4$ bis $R^8$ eine 1,1,4,4-Tetramethyl-1,4-butylenkette oder einen annellierten Benzolring bedeuten und |
| m, n | 0 bedeuten, |

mit der Maßgabe, daß m und n nicht für 0 stehen, wenn jeweils $R^6$ Halogen, $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten, dadurch gekennzeichnet, daß man ein Keton der Formel II

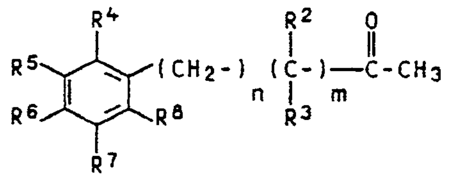 II

mit Distickstofftrioxid in Gegenwart von überschüssigem Alkohol der Formel $R^1$-OH und in Gegenwart eines sauren Katalysators bei einer Temperatur von 0 bis 140 °C behandelt.

**5.** Verfahren zur Herstellung von Dialkoxyketonen der Formel I nach Anspruch 1, in der

| | |
|---|---|
| $R^1$ | Methyl, |
| $R^2$, $R^3$ | Wasserstof oder $C_1$-$C_4$-Alkyl, |
| $R^4$-$R^8$ | Wasserstoff, Fluor, Chlor, Brom, Trifluormethyl, Trifluormethoxy oder zwei benachbarte Reste $R^4$ bis $R^8$ eine 1,1,4,4-Tetramethyl-1,4-butylenkette oder einen annellierten Benzolring bedeuten und |
| m, n | 0 bedeuten, |

mit der Maßgabe, daß m und n nicht für 0 stehen, wenn jeweils $R^6$ Halogen, $R^4$, $R^6$ und $R^8$ oder $R^4$, $R^6$ und $R^7$ oder $R^5$, $R^6$ und $R^8$ jeweils Halogen oder alle Reste $R^4$ bis $R^8$ Wasserstoff bedeuten, dadurch gekennzeichnet, daß man ein Keton der Formel 11

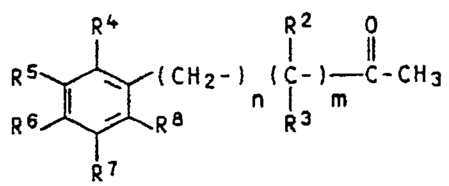 II

mit Distickstofftrioxid in Gegenwart von überschüssigem Alkohol der Formel $R^1$-OH und in Gegenwart

eines sauren Katalysators bei einer Temperatur von 0 bis 140 ° C behandelt.

## Claims

1. A process for the preparation of a dialkoxyketone of the formula I

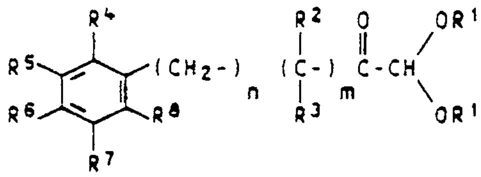

(I)

where $R^1$ is $C_1$-$C_{13}$-alkyl, $R^2$ and $R^3$ are each hydrogen or $C_1$-$C_4$-alkyl, $R^4$, $R^5$, $R^6$, $R^7$ and $R^8$ are each hydrogen, halogen, $C_1$-$C_4$-alkyl, $C_1$-$C_4$-haloalkyl, $C_1$-$C_4$-alkoxy, $C_1$-$C_4$-alkylthio, $C_1$-$C_4$-haloalkoxy, $C_1$-$C_4$-haloalkylthio, or phenyl which is unsubstituted or substituted by halogen, $C_1$-$C_4$-alkyl or $C_1$-$C_4$-alkoxy, or two adjacent radicals $R^4$ to $R^8$ form a $C_3$-$C_5$-alkylene chain which is unsubstituted or substituted by $C_1$-$C_4$-alkyl, or form an unsubstituted or substituted fused aromatic ring, and m and n are each 0 or 1, with the proviso that m and n are not 0, when $R^4$ or $R^8$ is $C_1$-$C_4$-alkoxy, $R^5$ or $R^7$ is $C_1$-$C_4$-alkylthio, $R^6$ is halogen, $C_1$-$C_4$-alkyl, $C_1$-$C_4$-alkoxy or phenyl, $R^5$ and $R^6$ or $R^6$ and $R^7$ are each $C_1$-$C_4$-alkoxy, $R^4$, $R^6$ and $R^8$ or $R^4$, $R^6$ and $R^7$ or $R^5$, $R^6$ and $R^8$ are each halogen or each of the radicals $R^4$ to $R^8$ is hydrogen, wherein a ketone of the formula II

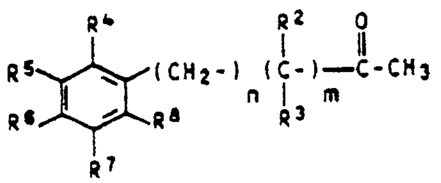

(II)

is treated with dinitrogen trioxide in the presence of an excess of an alcohol of the formula $R^1$-OH and in the presence of an acidic catalyst at from 0 to 140 ° C.

2. A process for the preparation of a dialkoxyketone of the formula I as claimed in claim 1, wherein $R^1$ is $C_1$-$C_5$-alkyl, $R^2$ and $R^3$ are each hydrogen or $C_1$-$C_4$-alkyl, $R^4$, $R^5$, $R^6$, $R^7$ and $R^8$ are each hydrogen, fluorine, chlorine, bromine, $C_1$-$C_4$-alkyl, $C_1$-$C_4$-fluoroalkyl, $C_1$-$C_4$-chloroalkyl, $C_1$-$C_4$-chlorofluoroalkyl, $C_1$-$C_4$-alkoxy, $C_1$-$C_4$-alkylthio, $C_1$-$C_4$-fluoroalkoxy, $C_1$-$C_4$-chloroalkoxy, $C_1$-$C_4$-chlorofluoroalkoxy, $C_1$-$C_4$-fluoroalkylthio, $C_1$-$C_4$-chloroalkylthio, $C_1$-$C_4$-chlorofluoroalkylthio, chlorophenyl, methylphenyl or $C_1$- or $C_2$-alkoxyphenyl, or two adjacent radicals $R^4$ to $R^8$ are each a $C_3$-$C_5$-alkylene chain which is unsubstituted or substituted by methyl, or are each a fused ring which is unsubstituted or substituted by halogen and/or $C,C_4$-alkyl and/or trifluoromethyl and/or $C_1$- or $C_2$-alkoxy and/or methylthio and/or trifluoromethoxy and/or trifluoromethylthio, and m and n are each 0 or 1, with the proviso that m and n are not 0 if $R^4$ or $R^8$ is $C_1$-$C_4$-alkoxy, $R^5$ or $R^7$ is $C_1$-$C_4$-alkylthio, $R^6$ is halogen, $C_1$–$C_4$-alkyl, $C_1$-$C_4$-alkoxy or phenyl, $R^5$ and $R^6$ or $R^6$ and $R^7$ are each $C_1$-$C_4$-alkoxy, $R^4$, $R^6$ and $R^8$ or $R^4$, $R^6$ and $R^7$ or $R^5$, $R^6$ and $R^8$ are each halogen, or each of the radicals $R^4$ to $R^8$ is hydrogen, wherein a Ketone of the formula II

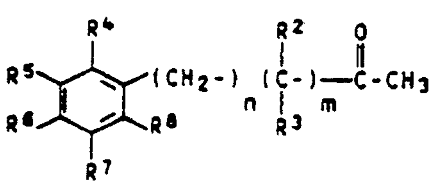

(II)

is treated with dinitrogen trioxide in the presence of an excess of an alcohol of the formula $R^1$-OH and in the presence of an acidic catalyst at from 0 to 140 °C.

3. A process for the preparation of a dialkoxyketone of the formula I as claimed in claim 1, wherein $R^1$ is $C_1$-$C_5$-alkyl, $R^2$ and $R^3$ are each hydrogen or $C_1$-$C_4$-alkyl, $R^4$, $R^5$, $R^6$, $R^7$ and $R^8$ are each hydrogen, fluorine, chlorine, bromine, $C_1$-$C_4$-alkyl, $C_1$-$C_4$-fluoroalkyl, $C_1$-$C_4$-chloroalkyl, $C_1$-$C_4$-chlorofluoroalkyl, $C_1$-$C_4$-alkoxy, $C_1$-$C_4$-alkylthio, $C_1$-$C_4$-fluoroalkoxy, $C_1$-$C_4$-chloroalkoxy, $C_1$-$C_4$-chlorofluoroalkoxy, $C_1$-$C_4$-fluoroalkylthio, $C_1$-$C_4$-chloroalkylthio, $C_1$-$C_4$-chlorofluoroalkylthio, chlorophenyl, methylphenyl or $C_1$- or $C_2$-alkoxyphenyl, or two adjacent radicals $R^4$ to $R^8$ are each a $C_3$-$C_5$-alkylene chain which is unsubstituted or substituted by methyl, or are each phenyl which is unsubstituted or substituted by halogen and/or $C_1$-$C_4$-alkyl and/or trifluoromethyl and/or $C_1$- or $C_2$-alkoxy and/or methylthio and/or trifluoromethoxy and/or trifluoromethylthio, or are each a fused benzene ring, and m and n are each 0 or 1, with the proviso that m and n are not 0 if $R^4$ or $R^8$ is $C_1$-$C_4$-alkoxy, $R^5$ or $R^7$ is $C_1$-$C_4$-alkylthio, $R^6$ is halogen, $C_1$-$C_4$-alkyl, $C_1$-$C_4$-alkoxy or phenyl, $R^5$ and $R^6$ or $R^6$ and $R^7$ are each $C_1$-$C_4$-alkoxy, $R^4$, $R^6$ and $R^8$ or $R^4$, $R^6$ and $R^7$ or $R^4$, $R^6$ and $R^8$ are each halogen, or each of the radicals $R^4$ to $R^8$ is hydrogen, wherein a ketone of the formula II

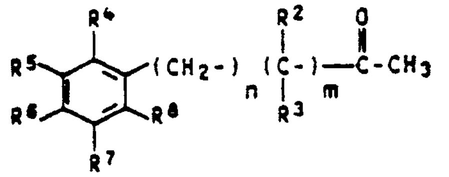
(II)

is treated with dinitrogen trioxide in the presence of an excess of an alcohol of the formula $R^1$-OH and in the presence of an acidic catalyst at from 0 to 140 °C.

4. A process for the preparation of a dialkoxyketone of the formula I as claimed in claim 1, wherein $R^1$ is $C_1$-$C_5$-alkyl, $R_2$ and $R_3$ are each hydrogen or $C_1$-$C_4$-alkyl, $R^4$, $R^5$, $R^6$, $R^7$ and $R^8$ are each hydrogen, fluorine, chlorine, bromine, trifluoromethyl or trifluoromethoxy, or two adjacent radicals $R^4$ to $R^8$ are each a 1,1,4,4-tetramethyl-1,4-butylene chain or a fused benzene ring, and m and n are each 0, with the proviso that m and n are not 0 if $R^6$ is halogen, $R^4$, $R^6$ and $R^8$ or $R^4$, $R^6$ and $R^7$ or $R^5$, $R^6$ and $R^8$ are each halogen or each of the radicals $R^4$ to $R^8$ is hydrogen, wherein a ketone of the formula II

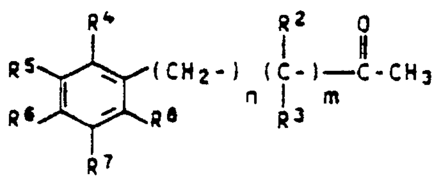
(II)

is treated with dinitrogen trioxide in the presence of an excess of an alcohol of the formula $R^1$-OH and in the presence of an acidic catalyst at from 0 to 140 °C.

5. A process for the preparation of a dialkoxyketone of the formula I as claimed in claim 1, wherein $R^1$ is methyl, $R^2$ and $R^3$ are each hydrogen or $C_1$-$C_4$-alkyl, $R^4$, $R^5$, $R^6$, $R^7$ and $R^8$ are each hydrogen, fluorine, chlorine, bromine, trifluoromethyl or trifluoromethoxy, or two adjacent radicals $R^4$ to $R^8$ are each a 1,1,4,4-tetramethyl-1,4-butylene chain or a fused benzene ring and m and n are each 0, with the proviso that m and n are not 0 if $R^6$ is halogen, $R^4$, $R^6$ and $R^8$ or $R^4$, $R^6$ and $R^7$ or $R^5$, $R^6$ and $R^8$ are each halogen or each of the radicals $R^4$ to $R^8$ is hydrogen, wherein a ketone of the formula II

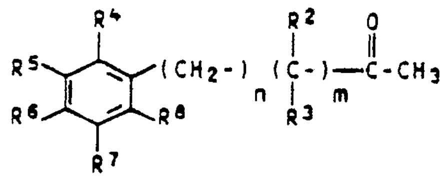

(II)

is treated with dinitrogen trioxide in the presence of an excess of an alcohol of the formula $R^1$-OH and in the presence of an acidic catalyst at from 0 to 140° C.

**Revendications**

1. Procédé de préparation de dialcoxycétones de formule I

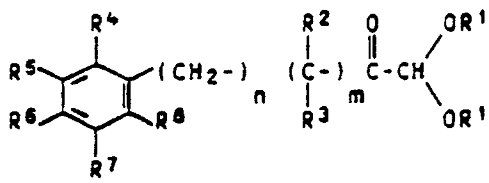

I

dans laquelle

| | |
|---|---|
| $R^1$ | represente un reste alkyle en $C_1$ à $C_{13}$, |
| $R^2$, $R^3$ | représentent chacun un atome d'hydrogène ou un reste $C_1$-$C_4$-alkyle, |
| $R^4$ - $R^8$ | représentent chacun un atome d'hydrogène, d'halogène, un reste $C_1$-$C_4$-alkyle, $C_1$-$C_4$-alkyle halogéné, $C_1$-$C_4$-alcoxy, $C_1$-$C_4$-alkylthio, $C_1$-$C_4$-alcoxy halogéné, $C_1$-$C_4$-alkylthio halogéné, phényle, qui est eventuellement substitue par un atome d'halogène, par un reste $C_1$-$C_4$-alkyle ou par un reste $C_1$-$C_4$-alcoxy; ou deux restes $R^4$ à $R^8$ voisins représentent une chaîne $C_3$-$C_5$-alkylène qui est éventuellement substituée par un reste $C_1$-$C_4$-alkyle, ou un noyau aromatique condensé éventuellement substitué, et |
| m, n | sont mis pour 0 ou 1, |

étant spécifié que m et n ne sont pas mis pour 0 chaque fois que $R^4$ ou $R^8$ représente un reste $C_1$-$C_4$-alcoxy; que $R^6$ ou $R^7$ représente un reste $C_1$-$C_4$-alkylthio; que $R^6$ représente un atome d'halogène, un reste $C_1$-$C_4$-alkyle, $C_1$-$C_4$-alcoxy, phényle; que $R^5$ et $R^6$ ou $R^6$ et $R^7$ représentent chacun un reste $C_1$-$C_4$-alcoxy; que $R^4$, $R^6$ et $R^8$ ou $R^4$, $R^6$ et $R^7$ ou $R^5$, $R^6$ et $R^8$ représentent chacun un atome d'halogène; ou que $R^4$ à $R^8$ sont tous des atomes d'hydrogène, caractérise en ce qu'on traite une cétone de formule II

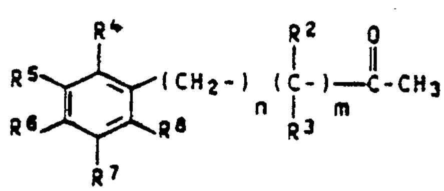

II

par de l'anhydride nitreux en présence d'un excès d'alcool de formule $R^1$OH et en présence d'un catalyseur acide, à une température de 0 à 140° C.

2. Procédé de préparation de dialcoxycétones de formule I selon la revendication 1, dans laquelle

| | |
|---|---|
| $R^1$ | représente un reste alkyle en $C_1$ à $C_5$, |
| $R^2$, $R^3$ | représentent chacun un atome d'hydrogène ou un reste $C_1$-$C_4$-alkyle, |
| $R^4$ - $R^8$ | représentent chacun un atome d'hydrogène, de fluor, de chlore, de brome, un reste $C_1$- |

$C_4$-alkyle, $C_1$-$C_4$-alkyle fluoré, $C_1$-$C_4$-alkyle chloré, $C_1$-$C_4$-alkyle chlorofluoré, $C_1$-$C_4$-alcoxy, $C_1$-$C_4$-alkylthio, $C_1$-$C_4$-alcoxy fluoré, $C_1$-$C_4$-alcoxy chloré, $C_1$-$C_4$-alcoxy chlorofluoré, $C_1$-$C_4$-alkylthio fluoré, $C_1$-$C_4$-alkylthio chloré, $C_1$-$C_4$-alkylthio chlorofluoré, chlorophényle, méthylphényle, $C_1$-$C_2$-alcoxyphényle; ou deux restes $R^4$ à $R^8$ voisins représentent une chaîne $C_3$-$C_5$-alkylène qui est éventuellement substituée par un reste méthyle, ou un noyau condensé éventuellement substitué par un atome d'halogène et/ou par un reste $C_1$-$C_4$-alkyle et/ou trifluorométhyle et/ou $C_1$-$C_2$-alcoxy et/ou méthylthio et/ou trifluorométhoxy et/ou trifluorométhylthio, et

m, n sont mis pour 0 au 1,

étant spécifié que m et n ne sont pas mis pour 0 chaque fois que $R^4$ au $R^8$ représente un reste $C_1$-$C_4$-alcoxy; que $R^5$ au $R^7$ représente un reste $C_1$-$C_4$-alkylthio; que $R^6$ représente un atome d'halogène, un reste $C_1$-$C_4$-alkyle, $C_1$-$C_4$-alcoxy, phényle; que $R^5$ et $R^6$ au $R^6$ et $R^7$ représentent chacun un reste $C_1$-$C_4$-alcoxy; que $R^4$, $R^6$ et $R^8$ au $R^4$, $R^6$ et $R^7$ au $R^5$, $R^6$ et $R^8$ représentent chacun un atome d'halogène; ou que $R^4$ à $R^8$ sont tous des atomes d'hydrogène, caractérisé en ce qu'on traite une cétone de formule II

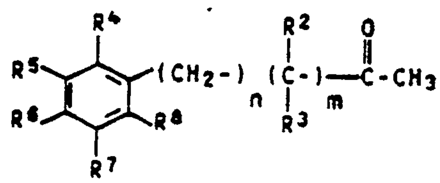

II

par de l'anhydride nitreux en présence d'un excès d'alcool de formule $R^1OH$ et en présence d'un catalyseur acide, à une température de 0 à 140 °C.

3. Procédé de préparation de dialcoxycétones de formule I selon la revendication 1, dans laquelle

$R^1$ représente un reste alkyle en $C_1$ à $C_5$,

$R^2$, $R^3$ représentent chacun un atome d'hydrogène au un reste $C_1$-$C_4$-alkyle,

$R^4$ - $R^8$ représentent chacun un atome d'hydrogène, de fluor, de chlore, de brome, un reste $C_1$-$C_4$-alkyle, $C_1$-$C_4$-alkyle fluoré, $C_1$-$C_4$-alkyle chloré, $C_1$-$C_4$-alkyle chlorofluoré, $C_1$-$C_4$-alcoxy, $C_1$-$C_4$-alkylthio, $C_1$-$C_4$-alcoxy fluoré, $C_1$-$C_4$-alcoxy chloré, $C_1$-$C_4$-alcoxy chlorofluoré, $C_1$-$C_4$-alkylthio fluoré, $C_1$-$C_4$-alkylthio chloré, $C_1$-$C_4$-alkylthio chlorofluoré, chlorophényle, methylphényle, $C_1$-$C_2$-alcoxyphényle; ou deux restes $R^4$ à $R^8$ voisins représentent une chaîne $C_3$-$C_5$-alkylène qui est éventuellement substituée par un reste méthyle, ou un radical phényle ou un noyeu benzénique condensé éventuellement substitué par un atome d'halogène et/ou par un reste $C_1$-$C_4$-alkyle et/au trifluorométhyle et/ou $C_1$-$C_2$-alcoxy et/ou méthylthio et/ou trifluorométhoxy et/ou trifluorométhylthio, et

m, n sont mis pour 0 au 1,

étant spécifié que m et n ne sont pas mis pour 0 chaque fois que $R^4$ ou $R^8$ représente un reste $C_1$-$C_4$-alcoxy; que $R^5$ ou $R^7$ représente un reste $C_1$-$C_4$-alkylthio; que $R^6$ représente un atome d'halogène, un reste $C_1$-$C_4$-alkyle, $C_1$-$C_4$-alcoxy, phényle; que $R^5$ et $R^6$ ou $R^6$ et $R^7$ représentent chacun un reste $C_1$-$C_4$-alcoxy; que $R^4$, $R^6$ et $R^8$ ou $R^4$, $R^6$ et $R^7$ ou $R^5$, $R^6$ et $R^8$ représentent chacun un atome d'halogène; ou que $R^4$ à $R^8$ sont tous des atomes d'hydrogène, caractérisé en ce qu'on traite une cétone de formule II

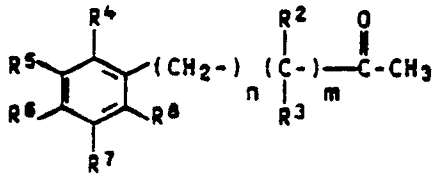

II

par de l'anhydride nitreux en présence d'un excès d'alcool de formule $R^1OH$ et en présence d'un catalyseur acide, à une température de 0 à 140 °C.

4. Procédé de préparation de dialcoxycétones de formule I selon la revendication 1,dans laquelle
   - $R^1$ représente un reste alkyle en $C_1$ à $C_5$,
   - $R^2$, $R^3$ représentent chacun un atome d'hydrogène ou un reste $C_1$-$C_4$-alkyle,
   - $R^4$ - $R^8$ représentent chacun un atome d'hydrogène, de fluor, de chlore, de brome, un reste trifluorométhyle, trifluorométhoxy; ou deux restes $R^4$ à $R^8$ voisins représentent une chaîne 1,1,4,4-tétraméthyl-1,4-butylène ou un noyau benzénique condensé, et
   - m, n sont mis pour 0,

   étant spécifié que m et n ne sont pas mis pour 0 chaque fois que $R^6$ représente un atome d'halogène, que $R^4$, $R^6$ et $R^8$ ou $R^4$, $R^6$ et $R^7$ ou $R^5$, $R^6$ et $R^8$ représentent chacun un atome d'halogène, ou que $R^4$ à $R^8$ sont tous des atomes d'hydrogène, caractérisé en ce qu'on traite une cétone de formule II

   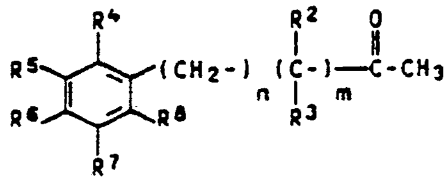 II

   par de l'anhydride nitreux en présence d'un excès d'alcool de formule $R^1OH$ et en présence d'un catalyseur acide, à une température de 0 à 140 °C.

5. Procédé de préparation de dialcoxycetones de formule I selon la revendication 1,dans laquelle
   - $R^1$ représente un reste méthyle,
   - $R^2$, $R^3$ représentent chacun un atome d'hydrogène ou un reste $C_1$-$C_4$-alkyle,
   - $R^4$ - $R^8$ représentent chacun un atome d'hydrogène, de fluor, de chlore, de brome, un reste trifluorométhyle, trifluorométhoxy, ou deux restes $R^4$ à $R^8$ voisins représentent une chaîne 1,1,4,4-tétraméthyl-1,4-butylène ou un noyau benzénique condensé, et
   - m, n sont mis pour 0,

   étant spécifié que m et n ne sont pas mis pour 0 chaque fois que $R^6$ représente un atome d'halogène, que $R^4$, $R^6$ et $R^8$ ou $R^4$, $R^6$ et $R^7$ ou $R^5$, $R^6$ et $R^8$ représentent chacun un atome d'halogène, ou que $R^4$ à $R^8$ sont tous des atomes d'hydrogène, caractérisé en ce qu'on traite une cétone de formule II

   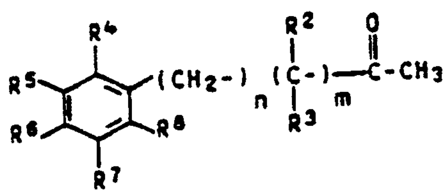 II

   par de l'anhydride nitreux en présence d'un excès d'alcool de formule $R^1OH$ et en présence d'un catalyseur acide, à une température de 0 à 140 °C.